# EUROPEAN PATENT APPLICATION

(11) **EP 2 138 167 A1**
(43) Date of publication of application: **30.12.2009**
(21) Application number: 08011444.0
(22) Date of filing: 24.06.2008
(51) Int. Cl.: A61K 9/48, A61K 31/403, A61K 9/16, C07D 403/06

(54) **Pharmaceutical composition comprising N-[2-(Diethylamino)ethyl]-5-[(5-fluoro-1,2-dihydro-2-oxo-3H-indol-3-ylidene) methyl]-2,4-dimethyl-1H-pyrrole-3-carboxamide**

(71) Applicant: Ratiopharm GmbH, 89070 Ulm (DE)
(72) Inventor: Ness, Winfried, 89231 Neu-Ulm (DE); Pätz, Jana, 86424 Dinkelscherben (DE); Brück, Sandra, 85570 Ottenhofen (DE); Muskulus, Frank, 82194 Gröbenzell (DE); Rimkus, Katrin, 24116 Kiel (DE)
(74) Representative: Teipel, Stephan

(57) **Abstract**

The present invention relates to a pharmaceutical composition comprising N-[2-(diethylamino)ethyl]-5-[(5-fluoro-1,2-dihydro-2-oxo-3H-indol-3-ylidene)methyl]-2,4-dimethyl-1 H-pyrrole-3-carboxamide as active pharmaceutical ingredient.

## Description

### Field of the invention

The present invention relates to a pharmaceutical composition comprising N-[2-(diethylamino)ethyl]-5-[(5-fluoro-1,2-dihydro-2-oxo-3H-indol-3-ylidene)methyl]-2,4-dimethyl-1 H-pyrrole-3-carboxamide as active pharmaceutical ingredient.

### Background of the invention

The invention relates to a pharmaceutical composition comprising N-[2-(Diethylamino)ethyl]-5-[(5-fluoro-1,2-dihydro-2-oxo-3H-indol-3-ylidene)methyl]-2,4-dimethyl-1 H-pyrrole-3-carboxamide and a process of preparing such composition.

The compound N-[2-(diethylamino)ethyl]-5-[(5-fluoro-1,2-dihydro-2-oxo-3H-indol-3-ylidene)methyl]-2,4-dimethyl-1 H-pyrrole-3-carboxamide (herein also referred to as Sunitinib) is a receptor tyrosine kinase inhibitor which is used to treat disorders like renal cell carcinoma (RCC) and gastrointestinal stromal tumors (GIST). Its activity relies on the inhibition of cellular signaling by targeting multiple receptor tyrosine kinases including platelet-derived growth factor receptors and vascular endothelial growth factor receptors. Since both kinds of receptors are involved in tumor angiogenesis and tumor cell proliferation, the simultaneous inhibition of these targets results in both reduced tumor vascularization and cancer cell death. These effects are responsible for the finally observed shrinkage of the renal cell carcinoma and gastrointestinal stromal tumors, respectively.

Sunitinib and its pharmaceutical effects on disorders like cancer are described in WO 01/60814. Further medical uses of Sunitinib and its salts are inter alia known from WO 01/45689 and WO 03/035009.

Typically, Sunitinib is administered in a dose of 50 mg once daily, which, if necessary, has to be varied according to individual tolerance and safety. Thus, in order to obtain sufficiently flexible dosing, individual dosage forms generally contain 12.5, 25 and 50 mg Sunitinib. Capsules comprising the malate salt of Sunitinib are sold under the brand name Sutent® (by Pfizer Pharma).

WO 01/60814 discloses two processes of preparing Sunitinib. According to these and other known manufacturing processes, Sunitinib is obtained as a solid.
One of the forms of Sunitinib is its crystalline malic acid salt as described in WO 03/016305.

The solid exhibits poor solubility in water affecting its oral bioavailability. Moreover, it has been reported to cause manufacturing problems (e.g. stickiness) when being processed in higher concentrations, especially concentrations over 40 wt.% (see for example WO 04/024127).

Apart from sufficient workability, good homogeneity and flowability of a pharmaceutical composition are further prerequisites for a successful manufacture of for example tablets and capsules on a production scale.

It is therefore an object of the invention to provide a pharmaceutical composition comprising Sunitinib which does not encounter the above problems. In particular the composition should possess improved properties such as solubility, homogeneity and flowability.

It has now been found that the above problems can be overcome by providing a pharmaceutical composition comprising crystalline Sunitinib in its free base form instead of e.g. its malate salt form or its amorphous form. This finding is particularly surprising because generally salt forms of organic pharmaceutically active compounds are more stable and exhibit a better processablity than their free base forms. Furthermore, amorphous forms of organic compounds are often better soluble than their crystalline forms. Therefore, a skilled person generally aims at providing a pharmaceutically active compound in an amorphous salt form. In contrast to this usual aim, for Sunitinib it has now been found that its crystalline free base form is more suitable for the preparation of a pharmaceutical composition.

Thus, the present invention relates to a pharmaceutical composition comprising crystalline N-[2-(diethylamino)ethyl]-5-[(5-fluoro-1,2-dihydro-2-oxo-3H-indol-3-ylidene)methyl]-2,4-dimethyl-1H-pyrrole-3-carboxamide as active pharmaceutical ingredient and at least one pharmaceutically acceptable excipient, wherein the active pharmaceutical ingredient is present in its free base form.

### Abbreviations

- A: Ångström
- DSC: differential scanning calorimetry
- DMSG: dynamic moisture sorption gravimetry
- IR: infrared
- RH: relative humidity
- RT: room temperature
- SCXRD: singe crystal X-ray diffraction
- XRPD: X-ray powder diffraction

### Brief description of the figures

Figure 1: XRPD pattern of Sunitinib in the polymorphic form I
Figure 2: XRPD pattern of Sunitinib in the polymorphic form II
Figure 3: XRPD pattern of Sunitinib in the polymorphic form III

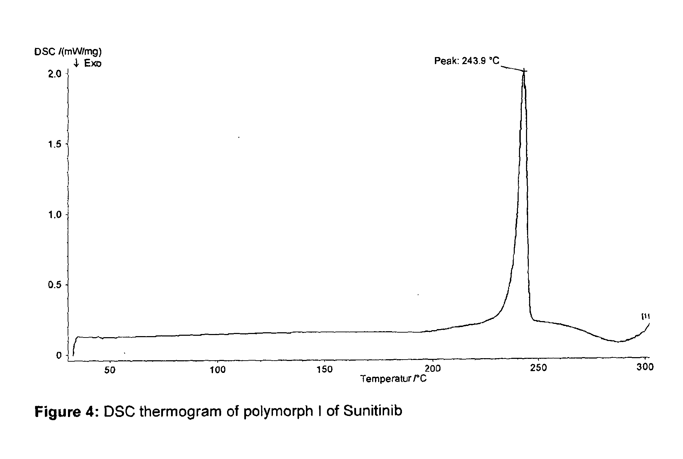

Figure 4: DSC diagram of Sunitinib in the polymorphic form I

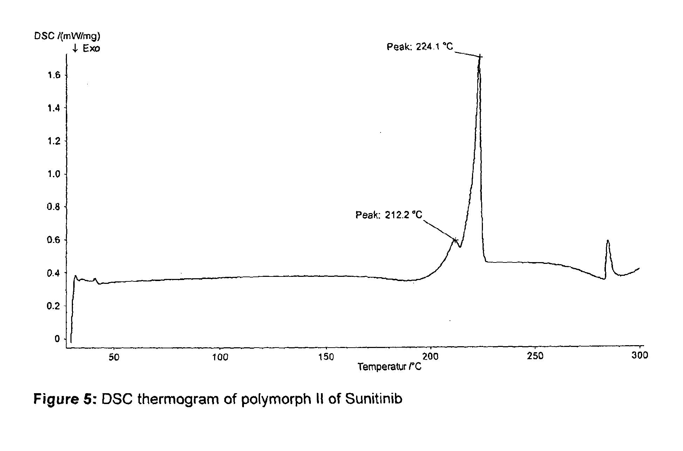

Figure 5: DSC diagram of Sunitinib in the polymorphic form II

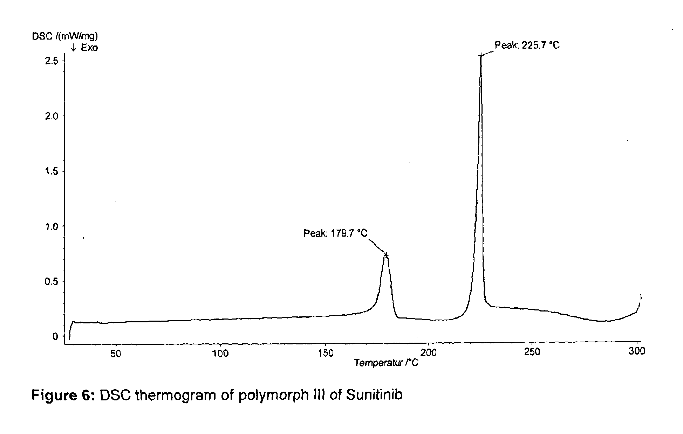

Figure 6: DSC diagram of Sunitinib in the polymorphic form III

### Description of the invention

N-[2-(diethylamino)ethyl]-5-[(5-fluoro-1,2-dihydro-2-oxo-3H-indol-3-ylidene)methyl]-2,4-dimethyl-1 H-pyrrole-3-carboxamide (Sunitinib) has the following chemical structure:

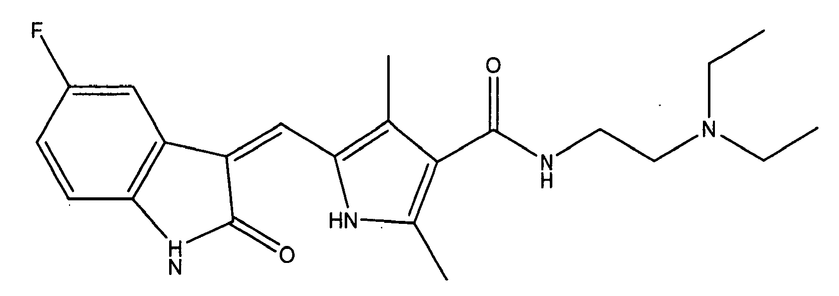

Sunitinib can be readily synthesized using techniques well known in the art. The synthesis of Sunitinib is disclosed for example in WO 01/60814.

The term "active pharmaceutical ingredient " (API) refers to crystalline Sunitinib in its free base form and to polymorphs, solvates or hydrates thereof.

The term "crystalline" refers to any non amorphous forms of the active pharmaceutical ingredient. The term "amorphous form" refers to a form of active pharmaceutical ingredient which has no long-range order like crystalline structures. The atoms or molecules of a material present in amorphous form are arranged in a non-uniform array. It is for example possible to distinguish amorphous from crystalline forms of a compound by powder X-ray diffraction.

It has been found that the above mentioned problems due to e.g. stickiness can be overcome by providing Sunitinib in its crystalline salt free form, preferably in its polymorphic form I, II and/or III which will be described in detail below. Due to the advantageous properties of crystalline Sunitinib in its free base form, the pharmaceutical composition of the present invention possesses excellent homogeneity and flowability as well as good solubility. Further, it shows convenient workability over the whole range of suitable concentrations of active pharmaceutical ingredient, i.e. about 5 to 80 % by weight based on the total weight of the composition.

Preferably, at least 60 % by weight, at least 70 % by weight or at least 80 % by weight of the active pharmaceutical ingredient are present in crystalline form, even more preferably at least 90 % by weight, most preferably at least 95 % by weight, wherein the respective amounts are being referred to the total weight of the active pharmaceutical ingredient in the composition of the present invention.

The term "pharmaceutical composition" refers to single dosage forms, such as tablets, capsules, pellets, etc., as well as powders or granules which are used in the preparation of single dosage forms. Where it is referred to the total weight of the pharmaceutical composition and the pharmaceutical composition in a single dosage form the total weight is the weight of the single dosage form excluding, if applicable, the weight of any coating or capsule shell.

The active pharmaceutical ingredient can be present in the pharmaceutical composition in an amount of 5 to 80 % by weight, preferably 10 to 70 % by weight, more preferably more than 40 to 60 % by weight of the total weight of the composition.

The active pharmaceutical ingredient is preferably present in its polymorphic form I, II and/or III, more preferably in its polymorphic form II and/or III, most preferably in its polymorphic form III.

### Polymorph I of Sunitinib

In one embodiment the pharmaceutical compositon of the present invention comprises a yellow to greenish yellow solid that will in the following be referred to as polymorph I of Sunitinib. It is characterized by an XRPD pattern having a characteristic peak at 4.5 ± 0.2 degrees 2-theta, in particular with peaks at 4.5 ± 0.2; 9.1 ± 0.2; 16.8 ± 0.2 and 26.0 ± 0.2 degrees 2-theta.

Furthermore, polymorph I of Sunitinib can be characterized by an XRPD pattern with peaks at 4.5 ± 0.2; 9.1 ± 0.2; 15.2 ± 0.2; 16.8 ± 0.2; 18.3 ± 0.2; 20.4 ± 0.2; 21.8 ± 0.2; 22.9 ± 0.2 and 26.0 ± 0.2 degrees 2-theta.

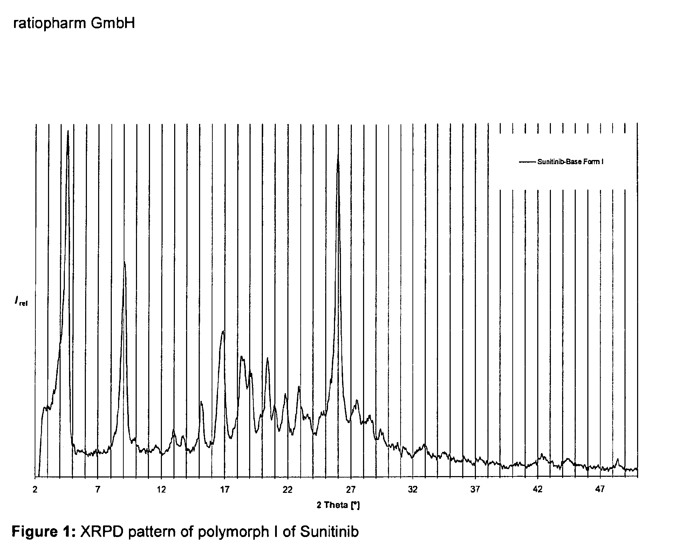

The XRPD pattern of polymorph I of Sunitinib is shown in figure 1.

Polymorph I of Sunitinib shows a Raman spectrum exhibiting characteristic peaks at 2927 ± 2 cm⁻¹, 1679 ± 2 cm⁻¹, 1585 ± 2 cm⁻¹, 1437 ± 2 cm⁻¹, 1334 ± 2 cm⁻¹, 1278 ± 2 cm⁻¹ and 670 ± 2 cm⁻¹_{.}

Polymorph I of Sunitinib shows an IR spectrum exhibiting characteristic peaks at 2969 ± 2 cm⁻¹, 1479 ± 2 cm⁻¹, 1330 ± 2 cm⁻¹, 1189 ± 2 cm⁻¹, 797 ± 2 cm⁻¹, 667 ± 2 cm⁻¹ and 609 ± 2 cm⁻¹.

Polymorph I of Sunitinib exhibits a low hygroscopicity. DMSG revealed a moisture sorption of maximum about 1 % referred to the weight of the sample.

### Polymorph II of Sunitinib

In the second embodiment the pharmaceutical composition of the present invention comprises polymorph II of Sunitinib. This crystal form has the following characteristics:
- crystal system triclinic
- space group P-1
- cell metrics a = 13.5 ± 0.2 Å
   b = 14.4 ± 0.2 Å
   c = 24.3 ± 0.2 Å
   α = 79 ± 1 °
   β = 81 ± 1 °
   γ = 89 ± 1 °
- cell volume V 4598.85 Å³
- molecules per unit cell 8

Polymorph II of Sunitinib is characterized by an XRPD pattern having a characteristic peak at 3.8 ± 0.2 degrees of 2-theta, in particular with peaks at 3.8 ± 0.2; 9.0 ± 0.2; 14.0 ± 0.2; 18.1 ± 0.2 and 20.5 ± 0.2 degrees 2-theta.

Furthermore, polymorph II of Sunitinib can be characterized by an XRPD pattern with peaks at 3.8 ± 0.2; 9.0 ± 0.2; 14.0 ± 0.2;18.1 ± 0.2; 20.5 ± 0.2; 26.6 ± 0.5 and 27.5 ± 0.6 degrees 2-theta.

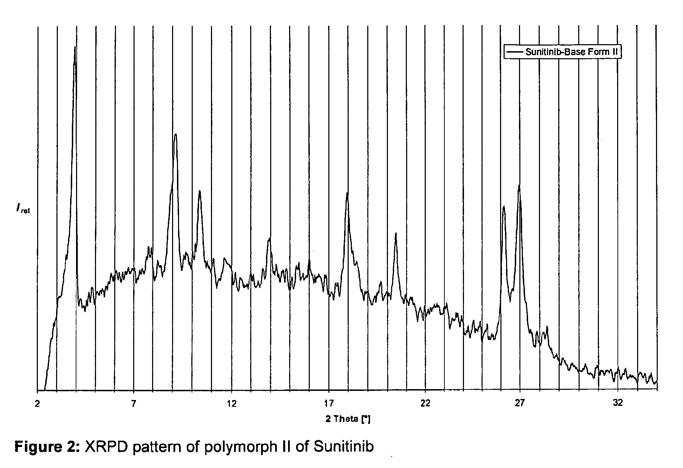

The XRPD of polymorph II of Sunitinib is shown in figure 2.

Polymorph II of Sunitinib shows a Raman spectrum exhibiting characteristic peaks at 2929 ± 2 cm⁻¹, 1627 ± 2 cm⁻¹, 1583 ± 2 cm⁻¹, 1425 ± 2 cm⁻¹, 1328 ± 2 cm⁻¹, 1285 ± 2 cm⁻¹, 1264 ± 2 cm⁻¹ and 669 ± 2 cm⁻¹.

Polymorph II of Sunitinib shows an IR spectrum exhibiting characteristic peaks at 1667 ± 2 cm⁻¹, 1476 ± 2 cm⁻¹, 1325 ± 2 cm⁻¹, 1147 ± 2 cm⁻¹, 794 ± 2 cm⁻¹, 668 ± 2 cm⁻¹ and 608 ± 2 cm⁻¹.

Sunitinib in the polymorphic form II exhibits some hygroscopicity. DMSG revealed a moisture sorption of more than 6 % referred to the weight of the sample.

Polymorph II of Sunitinib is obtainable by a process, comprising the steps of:
- dissolving Sunitinib present in any polymorphic or amorphous form or mixtures thereof, preferably in its polymorphic form I in a suitable inert solvent, preferably in water or an organic solvent or a mixture of two or more suitable inert solvents, preferably at a temperature between 25 °C and the reflux temperature of the solvent or the solvent mixture,
- optionally adding another solvent, preferably another organic solvent,
- concentrating the solution, preferably under reduced pressure, more preferably at a temperature between 25 °C and the reflux temperature of the solvent or the solvent mixture, most preferably under reduced pressure at 25 to 60 °C,
- collecting the resulting crystalline precipitate, preferably by filtration.

The crystallization is carried out in a suitable inert solvent or in a mixture of two or more suitable inert solvents. Examples of such suitable inert solvents are water, aliphatic and aromatic hydrocarbons (preferably hexane, benzene, toluene or xylene), aliphatic alcohols (preferably methanol, ethanol, propanol, iso-propanol), ethers (preferably diethyl ether, diisopropyl ether or dimethoxyethane), cyclic ethers (preferably tetrahydrofuran or dioxane), ketones (preferably acetone, methylisobutylketone or methylethylketone), esters (preferably ethylacetate), chlorinated hydrocharbons (preferably dichloromethane or chloroform) or nitrogen containing organic solvents (preferably N-methyl pyrollidone, dimethylformamide or acetonitrile). Acetone and toluene are especially preferred.

An alternative process for the preparation of polymorph II of Sunitinib comprises the steps of:
- dissolving a salt of Sunitinib, preferably a salt of Sunitinib (for example as disclosed in WO 01/060814), more preferably the malate salt, in a suitable inert solvent, preferably in water, or a mixture of two or more suitable inert solvents optionally heating the solvent to a temperature between 25 °C and the reflux temperature of the solvent or the solvent mixture,
- adding a base, preferably NaOH,
- collecting the resulting crystalline precipitate, preferably by filtration.

As solvents the suitable inert solvents as described above can be used.

The above described reaction is carried out in the presence of a base, preferably a Bronsted base. Examples of suitable Bronsted bases are metal hydroxides, metal carbonates or amines, preferably alkali metal hydroxides, alkali metal carbonates, alkali metal bicarbonates, ammonia, or organic amines, such as, for example, sodium hydroxide, potassium hydroxide, sodium carbonate, potassium carbonate, sodium bicarbonate, ammonia, diethylamine, triethylamine, diisopropylamine or pyridine. Sodium hydroxide is preferred. The base is used in an amount sufficient to obtain the free base of Sunitinib from its salt.

### Polymorph III of Sunitinib

In the third embodiment the pharmaceutical composition of the present invention comprises polymorph III of Sunitinib. This crystal form has the following characteristics:
- crystal system monoclinic
- space group P 2₁/n
- cell metrics a = 4.97560(10) Å
   b = 28.1365(6) Å
   c = 14.5880(3) Å
   β = 93.5130(10) °
- cell volume V 2038.42(7) Å³
- molecules per unit cell 4

Polymorph III of Sunitinib is characterized by an XRPD pattern having a characteristic peak at 6.3 ± 0.2 degrees 2-theta, in particular with peaks at 6.3 ± 0.2; 22.2 ± 0.2 and 26.4 ± 0.2 degrees 2-theta.

Furthermore, polymorph III of Sunitinib can be characterized by an XRPD pattern with peaks at 6.3 ± 0.2; 14.0 ± 0.2; 15.4 ± 0.2. 18.9 ± 0.2. 19.3 ± 0.2; 22.2 ± 0.2; 24.2 ± 0.2 and 26.4 ± 0.2 degrees 2-theta.

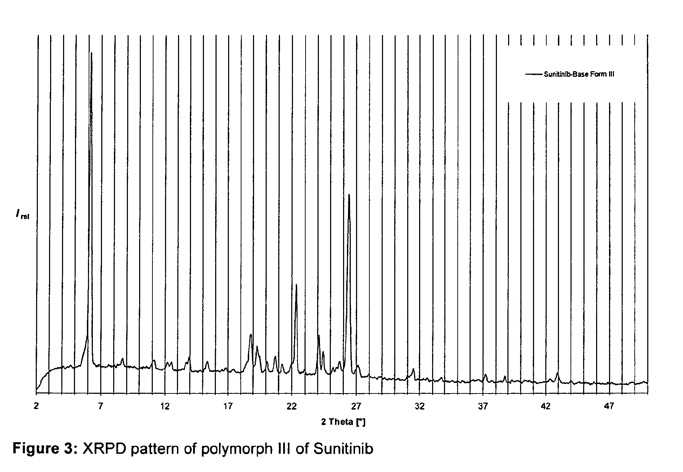

The XRPD pattern of polymorph III of Sunitinib is shown in figure 3.

Polymorph III of Sunitinib shows a Raman spectrum exhibiting characteristic peaks at 1674 t 2 cm⁻¹, 1569 ± 2 cm⁻¹, 1414 ± 2 cm⁻¹, 1327 ± 2 cm⁻¹, 1297 ± 2 cm⁻¹, 1259 ± 2 cm⁻¹, 1030 ± 2 cm⁻¹ and 666 ± 2 cm⁻¹.

Polymorph III of Sunitinib shows an IR spectrum exhibiting characteristic peaks at 3435 ± 2 cm⁻¹, 1670 ± 2 cm⁻¹, 1473 ± 2 cm⁻¹, 1294 ± 2 cm⁻¹, 1194 ± 2 cm⁻¹, 1146 ± 2 cm⁻¹, 786 ± 2 cm-¹, 663 ± 2 cm⁻¹, 617 ± 2 cm⁻¹.

Polymorph III exhibits a very low hygroscopicity. Under DMSG, the sample does not gain weight, even at a relative humidity of 95 %.

Polymorph III is preferably further characterized by the absence of any solvent molecules within the crystal.

A process for the preparation of polymorph III of Sunitinib comprises the steps of:
- preparing a clear saturated solution of Sunitinib in a suitable inert solvent,
- cooling the solution and allowing Sunitinib to crystallize,
- removing the resulting precipitate,
- keeping the filtrate at room temperature until Sunitinib crystallizes in the form of dark orange needles
or alternatively:
- preparing a clear saturated solution of Sunitinib in a suitable inert solvent,
- cooling the solution and seeding it with crystals of polymorph III of Sunitinib,
- allowing polymorph III to crystallize from the seeded solution at room temperature in the form of dark orange needles.

Preferably the process comprises the steps of:
- preparing a suspension of Sunitinib by suspending Sunitinib present in any polymorphic or amorphous form or mixtures thereof, preferably in its polymorphic form I or II, in a suitable inert solvent, preferably in an organic solvent or a mixture of two or more organic solvents,
- heating the suspension, preferably to the reflux temperature of the solvent or the solvent mixture, optionally by adding an additional amount of solvent, until a clear and saturated solution is obtained,
- cooling the solution and allowing Sunitinib to precipitate,
- removing the resulting precipitate, preferably by filtration,
- keeping the filtrate at room temperature until Sunitinib crystallizes in the form of dark orange needles,
or alternatively:
- preparing a suspension of Sunitinib by suspending Sunitinib present in any polymorphic or amorphous form or mixtures thereof, preferably in its polymorphic form I or II in a suitable inert solvent, preferably in an organic solvent or a mixture of two or more organic solvents,
- heating the saturated suspension, preferably to the reflux temperature of the solvent or the solvent mixture, optionally by adding an additional amount of solvent, until a clear and saturated solution is obtained,
- cooling the solution and seeding it with crystals of polymorph III of Sunitinib,
- allowing polymorph III of Sunitinib to crystallize from the seeded solution at room temperature in the form of dark orange needles.

The above described crystallizations are carried out in suitable inert solvents or mixtures of two or more suitable inert solvents. Examples of such suitable inert solvents are water, aliphatic and aromatic hydrocarbons (preferably hexane, benzene, toluene or xylene), aliphatic alcohols (preferably methanol, ethanol, propanol, iso-propanol), ethers (preferably diethyl ether, diisopropyl ether or dimethoxyethane), cyclic ethers (preferably tetrahydrofuran or dioxane), ketones (preferably acetone, methylisobutylketone or methylethylketone), esters (preferably ethylacetate), chlorinated hydrocharbons (preferably dichloromethane or chloroform) or nitrogen containing organic solvents (preferably N-methyl pyrollidone, dimethylformamide or acetonitrile). Acetone and toluene are especially preferred.

Advantageous properties regarding solubility, homogeneity and flowability are achieved if the pharmaceutical composition of the present invention has a mean particle size of 1 to 400 µm, preferably 10 to 200 µm, more preferably 20 to 100 µm.

A bulk density of the pharmaceutical composition ranging from of 0.3 to 0.85 g/ml, preferably of 0.3 to 0.6 g/ml, more preferably of 0.3 to 0.5 g/ml is advantageous.

The pharmaceutical composition of the invention preferably possesses Hausner ratios in the range of 1.05 to 1.65, more preferably of 1.1 to 1.5. The Hausner factor is the ratio of bulk density to tapped density.

The pharmaceutical composition of the present invention further comprises one or more pharmaceutically acceptable excipients, such as fillers, binding agents, lubricants, flow enhancers, antisticking agents, disintegrating agents and solubilizers. As pharmaceutically acceptable excipients conventional excipients known to the person skilled in the art may be used. See for example "Lexikon der Hilfsstoffe für Pharmazie, Kosmetik und angrenzende Gebiete", edited by H. P. Fiedler, 4th Edition, Edito Cantor, Aulendorf and earlier editions, and "Handbook of Pharmaceutical Excipients", Third Edition, edited by Arthur H. Kibbe, American Pharmaceutical Association, Washington, USA, and Pharmaceutical Press, London.

Preferred examples of the fillers are lactose, mannitol, sorbitol or microcrystalline cellulose. The filler is suitably present in an amount of 0 to 90 % by weight, preferably of 30 to 80 % by weight of the total weight of the composition.

The binding agent can for example be microcrystalline cellulose (MCC) or hydroxypropylmethyl cellulose (HPMC). Preferably the binding agent is present in an amount of 1 to 25 % by weight, more preferably at 2 to 10 % by weight of the total weight of the composition.

The lubricant is preferably a stearate, more preferably an earth alkali metal stearate, such as magnesium stearate. The lubricant is suitably present in an amount of 0.1 to 2 % by weight, preferably about 1 % by weight of the total weight of the composition.

Preferred disintegrating agents are croscarmellose sodium, sodium carboxymethyl starch or cross-linked polyvinylpyrrolidone (crospovidone). The disintegrating agent is suitably present in an amount of 0.1 to 20 % by weight, more preferably at about 0.5 to 7 % by weight of the total weight of the composition.

The flow enhancer can for example be colloidal silicon dioxide. Preferably, the flow enhancer is present in an amount of 0.5 to 8 % by weight, more preferably at 0.5 to 3 % by weight of the total weight of the composition.

The antisticking agent is for example talcum and may be present in amounts of 1 to 5 % by weight, more preferably in an amount of 1.5 to 3 % by weight of the total weight of the composition.

If desired, an improvement of the solubility of the active pharmaceutical ingredient can for example be achieved by the addition of complex forming agents/compounds (e.g. sodium benzoate, sodium salicylate or cyclodextrins), alternation of solvent properties (e.g. by adding PVP or polyethylene glycols) or the addition of solubilizers which form tenside micelles (e.g. surfactants).

Suitable solubilizers are for example surfactants such as polyoxyethylene alcohol ethers (e.g. Brij®), polysorbates (e.g. Tween®) or polyoxypropylene polyoxyethylene copolymers (poloxamer; e.g. Pluronic®) and may be present in amounts of 0.5 to 7 % by weight, more preferably 1 to 5 % by weight of the total weight of the composition.

Alternatively, a pseudo-emulsifier can be used. Its mechanism of action mainly relies on an enhancement of viscosity. However, pseudo-emulsifiers also possess emulsifying properties. Preferred pseudo-emulsifiers of the present invention are for example cellulose ethers, gum Arabic or tragacanth and may be present in amounts of 1 to 10 % by weight, more preferably 3 to 7 % by weight of the total weight of the composition.

A person skilled in the art may use these or other excipients in regard to the selected process of preparing the pharmaceutical composition of the invention.

The pharmaceutical composition of the present invention can be formulated in any known form, preferably as tablets, capsules, granules, pellets or sachets. A particularly preferred pharmaceutical composition is in the form of capsules. The pharmaceutical composition may contain dosage amounts of 12.5, 25 and 50 mg of the active pharmaceutical ingredient. Thus the administered amount can be readily varied according to individual tolerance and safety warranting more flexible dosing than the standard dose of 50 mg once daily.

The pharmaceutical composition of the present invention can be manufactured according to standard methods known in the art. Granulates according to the invention can be obtained by dry compaction or wet granulation. These granulates can subsequently be mixed with e.g. suitable disintegrating agents, glidants and lubricants and compressed into tablets or filled into sachets or capsules of suitable size. Tablets can also be obtained by direct compression of a suitable powder mixture, i.e. without any preceding granulation of the excipients. Suitable powder or granulate mixtures according to the invention are further obtainable by spray drying, lyophilization, melt extrusion, pellet layering, coating of the active pharmaceutical ingredient or any other suitable method provided that the conditions are chosen such as to prevent amorphization of the active pharmaceutical ingredient. The so obtained powders or granulates can be mixed with one or more suitable ingredients and the resulting mixtures can either be compressed to form tablets or filled into sachets or capsules. The above mentioned methods known in the art also include grinding and sieving techniques permitting the adjustment of desired particle size distributions.

The invention is further illustrated by the following examples which are not constructed as being limiting.

### Examples

### Example 1: preparation of polymorph II of Sunitinib

Sunitinib was obtained according to example 35 (alternative synthesis) of EP 1255752 B1. 0.25 g of Sunitinib were mixed with 50 ml acetone and heated to reflux. Then, 5 ml toluene were added. The solvent was removed at 45 °C under reduced pressure of 250 to 300 mmHg until precipitation of the crystalline solid occurred. The product was collected by filtration, washed with 2 ml of cold toluene and dried for 24 hours at room temperature.

### Example 2: preparation of polymorph III of Sunitinib

Sunitinib was obtained according to example 35 (alternative synthesis) of EP 1255752 B1. A suspension of Sunitinib was prepared in a mixture of acetone and toluene (1:1 v/v) at room temperature (RT). The suspension was heated to reflux for 1 hour, leading to a complete dissolution of Sunitinib. The solution was allowed to cool below boiling temperature and was then further cooled with a mixture of ice and sodium chloride as external cooling for 24 hours. The ice bath was not exchanged and the mixture was allowed to warm to RT. Further crystallization at RT for 48 hours resulted in a yellow precipitate, which was removed by filtration. The dark orange filtrate was kept at RT until dark orange needles were formed. These were isolated and dried for 24 hours at RT.

### Example 3: Pharmaceutical preparations

The amounts of active pharmaceutical ingredient and all excipients given in the tables below refer to the contents per single capsule.

### Example 3.1

| **ingredient** | **product, e.g.** | **function** | **amount [mg]** |
|---|---|---|---|
| Sunitinib | | API | 12.5 |
| MCC | Avicel® PH-102 | filler | 60 |
| PVP | Kollidon® 12.5 | binder | 10 |
| lactose+MCC (75:25) | MicroceLac®100 | filler | 20 |
| crospovidone | Kollidon® CL | disintegrant | 10 |
| magnesium stearate | | lubricant | 1 |
| colloidal silicon dioxide | Aerosil® 200 | flow enhancer | 0.5 |

Povidone is dissolved in a suitable solvent, e.g. water, and micronized Sunitinib is added to the solution. The resulting suspension is used to granulate MCC and Crospovidone. The dry granulate is pre-mixed with lactose+MCC and colloidal silicon dioxide and finally mixed with magnesium stearate. The mixture is filled into capsules.

### Example 3.2

| **ingredient** | **product, e.g.** | **function** | **amount [mg]** |
|---|---|---|---|
| Sunitinib micronized | | API | 50 |
| MCC | Avicel® PH-102 | filler | 20 |
| PVP | Kollidon® 90 | binder | 4 |
| crospovidone | Kollidon® CL | disintegrant | 8 |
| magnesium stearate | | lubricant | 1 |
| colloidal silicon dioxide | Aerosil® 200 | flow enhancer | 0.4 |

Povidone is dissolved in a suitable solvent, e.g. water, and 40 % of the micronized Sunitinib is added. The resulting suspension is used to granulate MCC, crospovidone and the residual amount of Sunitinib. The dry granulate is mixed with colloidal silicon dioxide and magnesium stearate and filled into capsules.

### Example 3.3

| **ingredient** | **product, e.g.** | **function** | **amount [mg]** |
|---|---|---|---|
| Sunitinib | | API | 25 |
| Sugar Spheres | Suglets® | carrier | 15 |
| HPMC | Klucel® | binder | 9 |
| talcum | | antisticking agent | 1 |

The sugar spheres are sprayed with an aqueous solution of API, HPMC and talcum (nozzle width 1 to 2 mm, inlet temperature 30 to 80 °C, product temperature about 35 to 40 °C). The dry coated pellets are filled into capsules.

### Example 3.4

| **ingredient** | **product, e.g.** | **function** | **amount [mg]** |
|---|---|---|---|
| Sunitinib | | API | 12.5 |
| anhydrous dicalcium phosphate | Di-Cafos® | filler | 42 |
| carageenan | Gelcarin® | binder | 7 |
| talcum | | antisticking agent | 1 |

Sunitinib, dicalcium phosphate and carrageenan are wet granulated, extruded and spheronized. The resulting pellets are mixed with talcum and filled into capsules.

### Example 3.5

| **ingredient** | **product, e.g.** | **function** | **amount [mg]** |
|---|---|---|---|
| Sunitinib | | API | 12.5 |
| MCC | Avicel® PH-101 | filler | 106 |
| PVP | Kollidon® 25 | binder | 10 |
| sodium croscarmellose | Ac-Di-SOL® | disintegrant | 9 |
| polysorbate | Tween®20 | solubilizer | 7.5 |
| magnesium stearate | | lubricant | 3 |
| colloidal silicon dioxide | Aerosil®200 | flow enhancer | 2 |

MCC, 5 g croscarmellose and 1 g colloidal silicon dioxide are mixed and fluid bed granulated with a suspension of Sunitinib, polysorbate and PVP in water. The granules are sieved and mixed with the residual amounts of croscarmellose and colloidal silicon dioxide and with magnesium stearate. The mixture is filled into capsules.

### Example 3.6

| **ingredient** | **product, e.g.** | **function** | **amount [mg]** |
|---|---|---|---|
| Sunitinib, micronized | | API | 25 |
| lactose | Granulac® | filler | 20 |
| hydroxypopylcellulose | Klucel® | binder | 3 |
| sodium croscarmellose | Ac-Di-SOL® | disintegrant | 5 |
| poloxamer | Pluronic® L81 | solubilizer | 2.5 |
| magnesium stearate | | lubricant | 1.5 |
| colloidal silicon dioxide | Aerosil® 200 | flow enhancer | 1 |

Sunitinib, lactose, 3 g sodium croscarmellose and 0.5 g colloidal silicon dioxide are mixed and fluid bed granulated with an aqueous solution of poloxamer and hydroxypropylcellulose. The granules are sieved and mixed with the residual amounts of croscarmellose and colloidal silicon dioxide and with magnesium stearate. The mixture is filled into capsules.

## Claims

1. Pharmaceutical composition comprising crystalline N-[2-(diethylamino)ethyl]-5-[(5-fluoro-1,2-dihydro-2-oxo-3H-indol-3-ylidene)methyl]-2,4-dimethyl-1 H-pyrrole-3-carboxamide in its free base form and at least one pharmaceutically acceptable excipient.

2. Pharmaceutical composition according to claim 1, wherein the crystalline N-[2-(di-ethylamino)ethyl]-5-[(5-fluoro-1,2-dihydro-2-oxo-3H-indol-3-ylidene)methyl]-2,4-dimethyl-1 H-pyrrole-3-carboxamide is present in its polymorphic form I, II and/or III.

3. Pharmaceutical composition according to claim 2, wherein the crystalline N-[2-(diethylamino)ethyl]-5-[(5-fluoro-1,2-dihydro-2-oxo-3H-indol-3-ylidene)methyl]-2,4-dimethyl-1 H-pyrrole-3-carboxamide is present in its polymorphic form III.

4. Pharmaceutical composition according to any of the preceding claims, wherein the active pharmaceutical ingredient is present in more than 40 to 60 % by weight of the total weight of the composition.

5. Pharmaceutical composition according to any of the preceding claims having a bulk density of 0.3 to 0.85 g/ml, preferably 0.3 to 0.6 g/ml, more preferably 0.3 to 0.5 g/ml.

6. Pharmaceutical composition according to any of the preceding claims having a mean particle size of 1 to 400 µm, preferably 10 to 200 µm, more preferably 20 to 100 µm.

7. Pharmaceutical composition according to any of the preceding claims having a Hausner ratio of 1.05 to 1.65, preferably 1.1 to 1.5.

8. Use of crystalline N-[2-(diethylamino)ethyl]-5-[(5-fluoro-1,2-dihydro-2-oxo-3H-indol-3-ylidene)methyl]-2,4-dimethyl-1H-pyrrole-3-carboxamide in its free base form for the manufacture of a pharmaceutical composition comprising at least one pharmaceutically acceptable excipient.
